# EUROPÄISCHE PATENTANMELDUNG

(11) **EP 1 745 788 A1**
(43) Veröffentlichungstag der Anmeldung: **24.01.2007**
(21) Anmeldenummer: 05106747.8
(22) Anmeldetag: 22.07.2005
(51) Int. Cl.: A61K 31/6615, A61P 25/28, A61P 35/00, A61P 3/00, A61P 29/00, A61P 43/00

(54) **Acylglycerophospholipide zur Behandlung von Krebs und Tumorkachexie**

(71) Anmelder: KTB Tumorforschungsgesellschaft mbH, 79106 Freiburg (DE)
(72) Erfinder: Massing, Ulrich, 79249 Merzhausen (DE)
(74) Vertreter: Helbing, Jörg

(57) **Zusammenfassung**

Die Erfindung betrifft die Verwendung von Acylglycerophospholipiden, insbesondere von hydrierten Acylglycerophospholipiden, zur Herstellung eines Medikaments zur Therapie von Krebs und Kachexie, Entzündungen und rheumatischen Erkrankungen, Fatigue, Polyneuropathie, multipler Sklerose, sowie zur Therapie der mit diesen Erkrankungen verbundenen Begleiterscheinungen, insbesondere zur Behandlung von Krebs und Tumorkachexie.

## Beschreibung

Die Erfindung betrifft die Verwendung von Acylglycerophospholipiden, insbesondere von hydrierten Acylglycerophospholipiden, zur Herstellung eines Medikaments zur Therapie von Krebs und Kachexie, Entzündungen und rheumatischen Erkrankungen, Fatigue, Polyneuropathie, multipler Sklerose, sowie zur Therapie der mit diesen Erkrankungen verbundenen Begleiterscheinungen, insbesondere zur Behandlung von Krebs und Tumorkachexie.

### Hintergrund der Erfindung

Phospholipide (nachfolgend kurz "PL") sind eine Hauptkomponente tierischer Zellmembranen. Sie bestehen in der Regel aus einem hydrophilen Kopf, der über eine negativ geladene Phosphatgruppe mit hydrophoben unpolaren Resten verknüpft ist. Die in biologischen Membranen häufigsten PL sind Glycerophospholipide.

Glycerophospholipide (nachfolgend "GPL") sind wie in Formel (I) gezeigt aufgebaut:

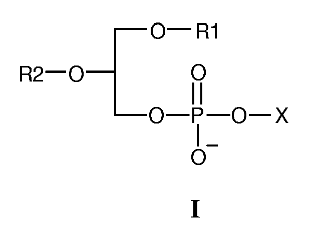

Sie bestehen aus einem hydrophilen Kopf, der über eine negativ geladene Phosphatgruppe in *sn*-3-Position mit einem Glycerinrückgrat und darüber mit einem oder zwei hydrophoben unpolaren Resten R¹ und R² verknüpft ist (IUPAC Compendium of Chemical Technology, 2nd ed. (1997)). Letztere sind in der Regel *O*-Acylreste aus Fettsäuren mit einer Länge von 14-24 C-Atomen, können jedoch auch *O*-Alkyl- oder *O*-1-Alkenylreste sein. GPL mit einem oder zwei *O-*Acylresten werden auch als 1-Acyl-, 2-Acyl- bzw. 1,2-Diacylglycerophospholipide bezeichnet oder pauschal als Acylglycerophospholipide (nachfolgend kurz "AGPL") zusammengefasst. Typische Acylglycerophospholipide sind Phosphatidylglycerin, Phosphatidylserin, Phosphatidylethanolamin (nachfolgend "PE"), Phosphatidylinositol (nachfolgend "PI"), Phosphatidsäure (nachfolgend "PA") und Phosphatidylcholin (nachfolgend "PC"). Acylglycerophospholipide sind insbesondere in Lecithin enthalten.

Glycerophospholipide, insbesondere PC oder PC-enthaltende Mischungen wie z. B. Lecithin, finden wegen ihrer Em ulgatorwirkung Einsatz als Bestandteil von Nahrungsergänzungsmitteln und Lebensmitteln, in der Kosmetik, in der parenteralen Ernährung und als Hilfsstoffe zur Formulierung von Arzneimitteln. Meist werden dabei nicht hydrierte Phospholipide aus Soja oder Ei verwendet, lediglich zur Formulierung von Medikamenten für die intravenöse Applikation werden hydrierte Phospholipide bevorzugt.

Eine tumorinhibierende Wirkung von Phospholipiden, welche nicht Acylglycerophospholipide sind, ist zwar in einigen Fällen bereits beschrieben, jedoch wird sie häufig von unerwünschten Nebenwirkungen insbesondere auf den Blutdruck und die Blutzusammensetzung begleitet (US 4,562,005; US 4,775,758; US 5,489,580; WO 01/72289; DE 4408011; Zeisig, R. et al., Anticancer Drug Des. 16(1):19-26 (2001); US 4,565,659). Ihr Einsatz als Wirkstoff in der Krebstherapie wurde dadurch bislang verhindert.

Als eine Alternative zu nativen Phospholipiden in der Tumortherapie wurden vor allem deren Alkylderivate, wie Alkylphosphocholine und Alkylphospholipide, oder Derivate mit anderen nicht-nativen Substituenten vorgeschlagen (US 4,562,005; US 4,775,758; EP 0171968; US 5,489,580; US 6,172,050; WO 01/72289; DE 440801 1 ; Zeisig, R. et al., Anticancer Drug Des. 16(1):19-26 (2001); Arndt, D. et al., Breast Cancer Res. Treat. 43(3):237-46 (1997); DE-A-3304870; US 4,492,659; DE-A-3204735; US 4,565,659; Modolell, M. et al., Cancer Res. 39(11):4681-4686 (1979)). Jedoch verhinderten die ausgeprägten Nebenwirkungen (insbesondere gastrointestinale Nebenwirkungen) die Gabe von zur Tumortherapie ausreichenden Mengen z. B. des vielversprechenden Alkylphosphocholins Hexadecylphosphocholin (Miltefosin) (Verveij, J. et al., J. Cancer Res. Clin. Oncol. 118:606 (1992)), so dass klinische Prüfungen mit der Substanz (orale Applikation) letztlich erfolglos blieben (Verveij, J et al., Eur. J. Cancer 29A:208 (1993); Planting, A.S. et al., Eur. J. Cancer 29A:518 (1993)). Miltefosin wird daher bis heute in onkologischen I ndikationen nur als Hautsalbe zur Behandlung von Hautmetastasen des Mammakarzinoms eingesetzt wird (Miltex^{®}) (Dummer, R. et al., J. Am. Acad. Dermatol. 29:963 (1993)).

Eine Applikation von Miltefosin per Injektion wurde bisher wegen spontaner Hämolyse nicht für eine Anwendung am Menschen in Betracht gezogen. Die biophysikalischen Eigenschaften von Miltefonsin entsprechen denen von Lyso-Phospholipiden, die bei lokal hohen Konzentrationen membranolytische Eigenschaften aufweisen.

Ähnliches gilt für Lyso-Phospholipide. Ein Lyso-Phospholipid ist ein durch Phospholipase A-katalysierte Abspaltung oder durch chemische Hydrolyse eines Fettsäurerestes entstehendes, einkettiges Phospholipid mit oberflächenaktiven Eigenschaften, welche rote Blutkörperchen zu lysieren vermögen (daher der Name). Lyso-Phospholipide (1-Acyl- oder 2-Acyl-glycerophospholipide) können aus den verschiedenen zweikettigen, membranbildenden Phospholipiden entstehen. Beispiele für Lyso-Phospholipide sind Lyso-Phosphatidylcholin, Lyso-Phosphatidylethanolamin (Lyso-Kephalin), Lyso-Phosphatidylglycerol, -serin und Lyso-Phosphatidsäure, deren Ausgangs-Phospholipide sämtlich 1,2-Diacylglycerophospholipide sind. Lyso-Phospholipide sind zwar als karzinostatische Verbindungen bekannt (US 4,372,949), haben aber wegen ihrer oberflächenaktiven Eigenschaften bei Applikation der reinen Lyso-Phospholipide in relevanten Dosen ebenso hämolytische Eigenschaften wie die oben diskutierten Phospholipide, die nicht Acylglycerophospholipide sind (US 4,562,005; US 4,775,758; EP 0171968; US 5,489,580; US 6,172,050; WO 01/72289; DE 4408011; Zeisig, R. et al., Anticancer Drug Des. 16(1):19-26 (2001); Arndt, D. et al., Breast Cancer Res. Treat. 43(3):237-46 (1997); DE-A-3304870; US 4,492,659; DE-A-3204735; US 4,565,659; Modolell, M. et al., Cancer Res. 39(11):4681-4686 (1979)).

Zudem kamen bislang weder Phospholipide noch Lysophospholipide zur Therapie von tumorinduzierter Kachexie und von Nierenzellkarzinomen zu Einsatz.

Glycerophospholipiden, welche üblicherweise zur Liposomenherstellung verwendet werden, wie z. B. Phosphatidylcholin, wird ein eigener antineoplastischer Effekt sogar abgesprochen (DE-A-19959689).

Phospholipide finden Verwendung als Trägerstoffe und Formulierhilfen (Emulgatoren/Vesikelbildner) für Arzneimittel z. B. in Liposomen. Dennoch ist bislang kein antitumoraler Effekt der Phospholipide selbst beschrieben. Somit beschränkt sich der Einsatz von Acylglycerophospholipiden in der Tumortherapie bislang auf ihren Einsatz als Trägerstoffe zur Formulierung von Wirkstoffen z. B. in wirkstoffbeladenen Liposomen (WO 99/49716).

Des weiteren werden Phospholipide zur Formulierung von Zubereitungen zur parenteralen Ernährung verwendet. Derartige Zubereitungen sind Emulsionen und bestehen typischerweise zu 10, 20 oder 30 % aus Triglyceriden (z. B. Sojaöl, MCT (medium chain triglyceride, mittelkettige Triglyceride), Olivenöl, Fischöle oder deren Mischungen (z. B. Soja/Olive 4:1)). Die Emulsionen enthalten des weiteren einen geringen Anteil Phospholipide (Lecithin) aus Hühnerei als Emulgatoren, wobei ein möglichst geringes Phospholipid/Triglycerid-Verhältnis angestrebt wird (z. B. Intralipid, Baxter, 10 % (20 %; 30 %) Emulsion: 10 g (20 g; 30 g) Sojaöl und 0,6 g (1,2 g; 1,2 g) Phospholipid/100 ml; Lipundin 10 % N, Braun: 8 g Lecithin auf 100 g Sojaöl; Lipofundin MCT 10%, Braun: 8 g Lecithin auf 100g Soja/MCT (1:1)). Emulsionen mit höherem Triglyceridgehalt benötigen zur Emulgation offensichtlich weniger Phospholipid, was als vorteilhaft angesehen wird. Bei niedrigem Phospholipid/Trigycerid-Verhältnis zeigt sich eine bessere metabolische Verträglichkeit mit signifikant geringerer Akkumulation von Phospholipiden und Cholesterol im Plasma (Hartig, W. et al. (Hrsg.), Ernährungs- und Infusionstherapie, 8. Aufl., Thieme (2004)). Die verwendeten Ei-Lecithine sind hochangereicherte Lecithine mit einem Glycerophosphatidylcholin-Anteil von typischerweise 75 oder 80 % (z. B. Lipoid E 75/E 80).

Als Tumorkachexie bzw. tumorinduzierte Kachexie bezeichnet man ein bei Tumorpatienten häufiges Syndrom mit hochgradiger Gewichtsabnahme und Änderungen der Körperzusammensetzung. Es kommt zum Abbau des Fettgewebes, der Skelettmuskulatur und zur Beeinträchtigung immunologischer Abwehrmechanismen (Tisdale, M.J., Nutrition 17(5):438-442 (2001); Tisdale, M.J., Curr. Opin. Clin. Nutr. Metab. Care 5(4):401-405 (2002)).

De Wys et al. berichteten, dass ein Gewichtsverlust der Tumordiagnose - abhängig von der Tumorentität - in 31-87% aller Fälle vorausgeht (DeWys, W.D. et al., Am. J. Med., 491 (1980)). Ein schwerer Gewichtsverlust von > 10% des gesunden Ausgangsgewichts ließ sich bei 15% aller Patienten bei Diagnosestellung feststellen. Tumortherapien sind mit weiterem Appetit- und Gewichtsverlust assoziiert (Costa, G. und Donaldson, S.S., N. Engl. J. Med., 1471 (1979)).

Die tumorinduzierte Kachexie ist ein schwerwiegendes Problem bei der Behandlung vieler Krebspatienten. Längsschnittstudien zeigten, daß Tumorpatienten mit zurückliegendem Gewichtsverlust eine schlechtere Prognose haben als solche mit stabilem Gewicht. Trotz stärkerer unerwünschter Therapiewirkungen ist das Tumoransprechen bei diesen Patienten geringer, ebenso finden sich eine reduzierte Leistungsfähigkeit, eine geringere Bewertung der subjektiven Lebensqualität und ein vermindertes Überleben (DeWys, W.D. et al., Am. J. Med., 491 (1980); Andreyev, H.J.H.,Eur. J. Cancer, 503 (1998); van Eys, J., Cancer Res., 747 (1982)). Neben der Sepsis ist die Kachexie eine häufige direkte Todesursache bei Tumorpatienten; Zahlenangaben schwanken zwischen 5 und 25% (Klastersky, J., Eur. J. Cancer, 149 (1972)).

Pfitzenmaier et al. berichteten, dass 60-70% aller Patienten mit fortgeschrittenem Prostatakarzinom an einer Kachexie leiden (Pfitzenmaier, J. et al., Cancer 97:1211 (2003)).

Die tumorinduzierte Kachexie kann als entzündlicher Prozess gesehen werden, da bei vielen der Patienten neben der Mangelernährung und dem Gewichtsverlust gleichzeitig erhöhte Konzentrationen von Entzündungsmarkern zu beobachten sind (Moldawer, F.F. und Copeland, E.M., Cancer, 1828 (1997)). Die beobachtete Freisetzung von Zytokinen, katabolen Hormonen und weiteren regulatorischen Peptiden scheint zunächst die primäre Reaktion der Wirtsgewebe des Tumorpatienten zu sein (Tisdale, M.J., Science, 2293 (2000)). Zusätzlich können weitere von Tumorzellen freigesetzte Substanzen, wie der "tumor lipid mobilizing factor" (LMF) und der "Proteolyse-induzierende Faktor" (PIF) katabole Signale beitragen und die Zytokinproduktion sowie die Akutphasenreaktion stimulieren (Tisdale, M.J., Science, 2293 (2000)).

Diese systemische Entzündungsreaktion wird heute als Ursache des beobachteten Verlustes von Appetit (Inui, A., Cancer Res., 4493 (1999)) und Körpergewicht (Fearon, K.C.H., World J. Surgery, 584 (1999)) gesehen, die parallel die Tumorprogression fördern kann (Coussens, L.M. und Werb, Z., Nature, 860 (2002)). Zytokin-induzierte metabolische Veränderungen scheinen bei kachektischen Patienten einen Wiederaufbau verlorener Körperzellmasse selbst bei künstlicher Ernährung zu verhindern (Espat, N.J., J. Surg. Oncol. 77, (1995)) und sind mit einer reduzierten Lebenserwartung assoziiert (O'Gormann, P. et. al., Nutrion and Cancer 36 (2000)). Das resultierende Syndrom aus vermindertem Appetit, Gewichtsverlust und Entzündungszustand wird als Kachexie, Tumorkachexie oder Anorexie-Kachexie-Syndrom bezeichnet (cancer anorexia-cachexia syndrome, CACS) (Nelson, K.A., Semin. Oncol., 64 (2000)).

Die der Erfindung zugrundeliegende Aufgabe war es somit, ein Medikament zu entwickeln, das ein Phospholipid als Wirkstoff enthält und zur Therapie von Krebs und Tumorkachexie geeignet ist.

### Kurzbeschreibung der Erfindung

Es wurde nun gefunden, dass Acylglycerophospholipide, insbesondere hydrierte Acylglycerophospholipide und vor allem hydriertes PC, antitumorale Wirkung haben und tumorinduzierte Kachexie verringern.

Die Erfindung umfasst somit
(1) die Verwendung von einem oder mehreren Acylglycerophospholipiden als Wirkstoff zur Herstellung eines Medikaments zur Therapie von Krebs und Kachexie, Entzündungen und rheumatischen Erkrankungen, Fatigue, Polyneuropathie, multipler Sklerose, sowie zur Therapie der mit diesen Erkrankungen verbundenen Begleiterscheinungen;
(2) eine bevorzugten Ausführungsform von (1), wobei das Acylglycerophospholipid nur gesättigte Acylreste besitzt und insbesondere ein Phosphatidylcholin mit gesättigten Acylresten ist; und
(3) eine weitere bevorzugte Ausführungsform von (1) und (2), in der das Medikament zur Behandlung von Krebs und Tumorkachexie geeignet ist.

### Kurzbeschreibung der Figuren

Die Erfindung wird anhand der nachfolgenden Figuren in der detaillierten Beschreibung der Erfindung näher erläutert.

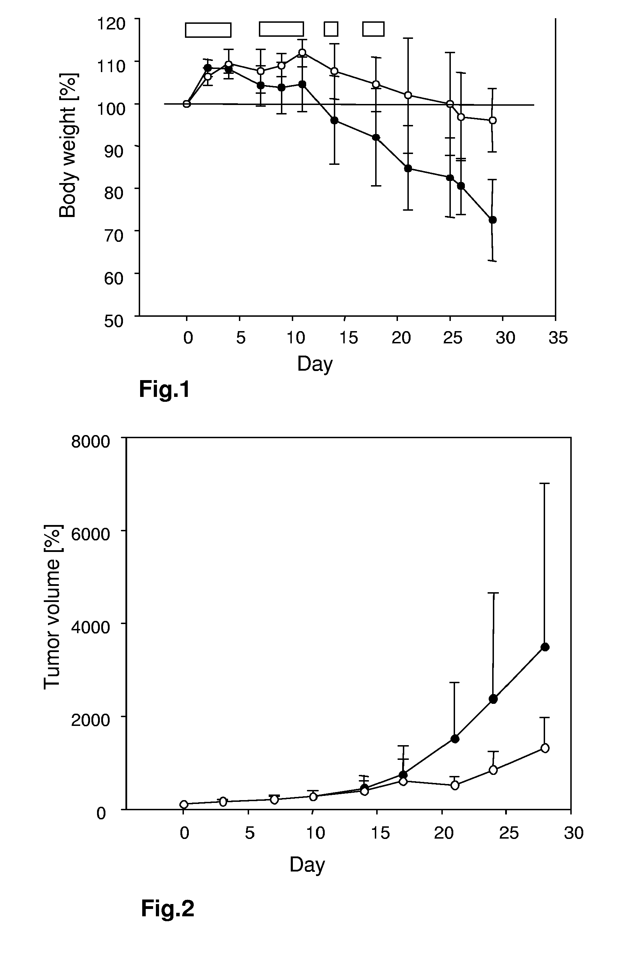

- Fig. 1:: Wirkung von Dipalmitoylphosphatidylcholin auf tumorinduzierte Kachexie bei Nacktmäusen, die ein humanes Nierenzellkarzinom (RXF 486) tragen. Punkte: Kontrolle ohne Lipid (n = 3); Kreise: 33 mg DPPC/kg/Tag (Tag 0-4, 7 - 11, 14, 17, 18, orale Applikation (n=3); Rechtecke definieren die Tage, an denen DPPC verabreicht wurde; p: < 0,001.
- Fig. 2:: Wirkung von hydriertem PC auf Tumorprogression bei humanen Weichteilsarkom auf der Nacktmaus. Punkte: Kontrolle ohne Lipid; Kreise: 840 mg EPC-3/kg/Woche (verabreicht an Tag 0, 7 und 14 durch i.v. Applikation in die Schwanzvene; n = 6).

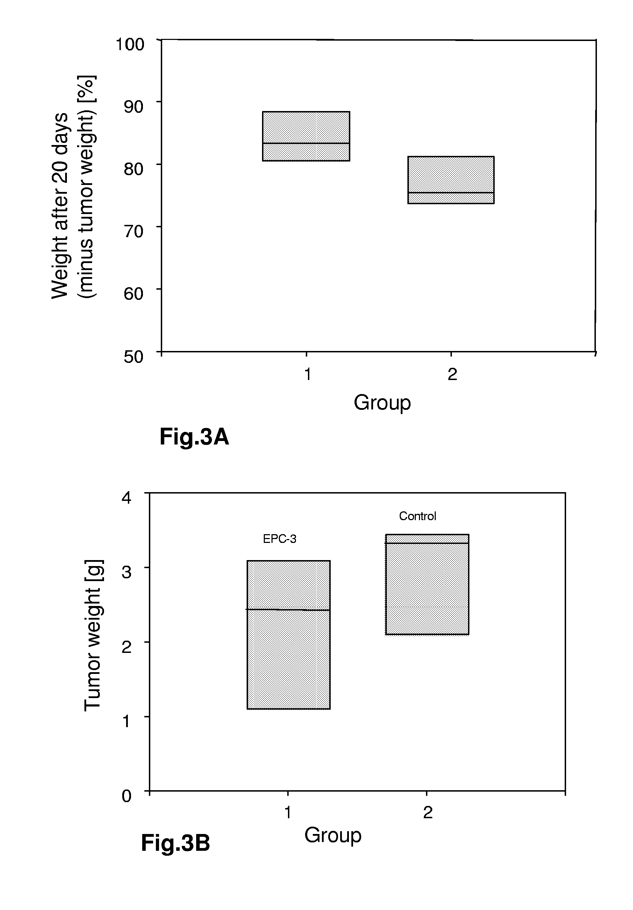

- Fig. 3:: Wirkung von hydriertem PC auf tumorinduzierte Kachexie und Tumor-gewicht in immunkompetenten Mäusen, die ein Nierenzell karzinom (RENCA-Modell, orthotopes Modell) tragen. Gruppe 1: 50 mg/kg/Tag EPC-3 (n = 5); Gruppe 2: unbehandelte Kontrolle (n = 6).
A) Gewicht. Gruppe 1: 84,3±4,3% des Ausgangsgewichtes, Gruppe 2: 76,9 ± 4,2 % des Ausgangsgewichtes.
B) Tumorgewicht (p: 0,227).

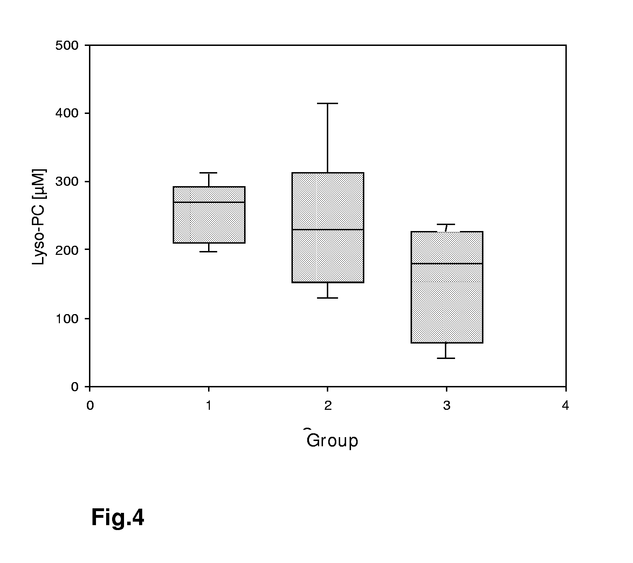

- Fig. 4:: Lyso-Phosphatidylcholin-Spiegel im Serum von Tumorpatienten mit und ohne Tumorkachexie. Gruppe 1: Tumorpatienten ohne Gewichtsverlust seit Erstdiagnose; Gruppe 2: Tumorpatienten mit weniger als 10 % Gewichtsverlust seit Erstdiagnose; Gruppe 3: Tumorpatienten mit mehr als 10 % Gewichtsverlust seit Erstdiagnose (stark kachektisch).

### Detaillierte Beschreibung der Erfindung

Die erfindungsgemäße Verwendung von APGL als Wirkstoff in einem Medikament hat sich als eine überraschend wirksame Methode zur Therapie von Krebs und Kachexie, Entzündungen und rheumatischen Erkrankungen, Fatigue, Polyneuropathie, multipler Sklerose, sowie zur Therapie der mit diesen Erkrankungen verbundenen Begleiterscheinungen herausgestellt. So zeigt die vorliegende Erfindung insbesondere, dass die Gabe von AGPL das Tumorwachstum und die Tumorkachexie verringern kann.

### Im folgenden werden zunächst einige der verwendeten Begriffe näher definiert:

Ein "Acylglycerophospholipid" ("AGPL") im Sinne der vorliegenden Erfindung ist z. B. ein 1 ,2-Diacylglycerophospholipid, 1-Acylglycerophospholipid oder 2-Acylglycerophospholipid mit gesättigten oder ungesättigten Acylresten, einschließlich Phosphatidylcholin, Lyso-Phosphatidylcholin und Lecithin, oder deren pharmazeutisch geeigneten Salzen. AGPL besitzt vorzugsweise die Struktur der Formel (I) 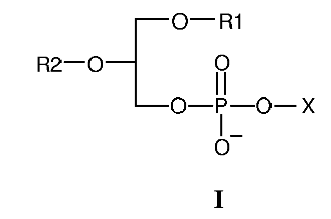 worin
R¹ und R² unabhängig voneinander ausgewählt sind aus H, Alkylcarbonyl-, Alkenylcarbonyl-, Alkinylcarbonyl-, Arylalkylcarbonyl- und Cycloalkylcarbonylresten, worin die Alkylreste geradkettig, verzweigt oder zyklisch, gesättigt oder ungesättigt und mit 1 bis 3 Resten R³ substituiert sein können und in den Alkylresten eines oder mehrere der C-Atome durch O oder NR⁴ ersetzt sein können;
X ausgewählt ist aus H (die Verbindung ist dann eine PA), (CH₂)ₙ-N(R⁴)₃⁺ (diese Verbindungsklasse umfasst PE und PC), -(CH₂)ₙ-CH(N(R⁴)₃⁺)-COO⁻ (diese Verbindunsklasse umfasst PS) und -(CH₂)ₙ-CH(OH)-CH2OH (diese Verbindungsklasse umfasst PG), wobei n eine ganze Zahl von 1 bis 5 ist;
R³ unabhängig vom Auftreten weiterer R³-Reste ausgewählt ist aus H, Niederalkyl (worin die Niederalkylreste geradkettig, verzweigt oder zyklisch, gesättigt oder ungesättigt sein können), F, Cl, CN und OH; und
R⁴ unabhängig vom Auftreten weiterer R⁴-Reste ausgewählt ist aus H, CH₃ und CH₂CH₃,
oder ein pharmakologisch geeignetes Salz desselben.

Die Acylreste sind bevorzugt Alkylcarbonylreste. Diese können gesättigt oder ungesättigt und gleich oder unterschiedlich lang sein, bevorzugt sind Kettenlängen von C10 bis C24, besonders bevorzugt Kettenlängen von C14 bis C22. Ungesättigte Alkylcarbonylreste sind bevorzugt ausgewählt aus ω-3 und ω-9 Fettsäuren, insbesondere aus Ölsäure (18:1), α-Linolensäure (18:3), Eicosapentaensäure (20:5) und Docosahexaensäure (22:6). Besonders bevorzugt sind AGPL, deren Acylreste gesättigt sind.

Für den erfindungsgemäßen Einsatz bevorzugt sind AGPL mit natürlich vorkommenden Kopfgruppen, insbesondere Phosphatidylcholine, Phosphatidylethanolamine, Phosphatidylglycerine, Phosphatidylserine und Phosphatidsäuren, ganz besonders APGL ausgewählt aus der Gruppe der Phosphatidylcholine. Besonders bevorzugt sind AGPL, welche ausschließlich gesättigte Acylreste besitzen, insbesondere Phosphatidylcholine mit gesättigten Acylresten.

I n einer besonders bevorzugten Ausführungsform von (1) werden Verbindungen mit der Struktur der Formel (1) oder deren pharmazeutisch geeignete Salze verwendet,
(i) die Alkylcarbonylreste 10 bis 24 C-Atome aufweisen, gesättigt sind oder eine oder mehrere Doppelbindungen enthalten, wobei die Zahl der C-Atome bevorzugt ein Vielfaches von 2 ist und die Doppelbindungen nicht konjugiert sind, und wobei die Alkylreste besonders bevorzugt Fettsäurereste sind;
(ii) die Niederalkylreste 1-3 C-Atome aufweisen und bevorzugt gesättigt sind; und
(iii) n eine ganze Zahl von 1 bis 3 ist.

Ganz besonders bevorzugt sind Verbindungen der Formel (I), worin
(i) R¹ und R² unabhängig voneinander H oder unverzweigte und unsubstituierte Alkylcarbonylreste sind, welche entweder gesättigt sind und dann bevorzugt ausgewählt sind aus Lauryl- (n-Dodecanyl-), Myristyl- (n-Tetradecanyl-), Palmitoyl- (n-Hexadecanyl-), Stearyl- (n-Octadecanyl-), Arachinyl- (n-Eicosanyl-), Behenyl- (n-Docosanyl-) und Lignoceryl- (n-Tetracosanyl-)resten und ganz besonders bevorzugt aus Myristyl-, Palmitoyl-, Stearyl- und Arachniylresten, oder ungesättigt sind und dann bevorzugt ausgewählt sind aus Oleyl- (18:1), α-Linolenyl- (18:3), Eicosapentaenyl- (20:5) und Docosahexaenyl (22:6)-Resten;
(ii) R³ H ist; und
(iii) X -(CH₂-)₂-N(CH₃)₃⁺, -(CH₂-)₂-NH₃⁺, oder -CH₂-CH(NH₃⁺)-COO⁻ ist.

Die Herkunft der AGPL (synthetisch oder aus natürlichen Quellen isoliert) ist für ihren erfindungsgemäßen Einsatz irrelevant. Auch hydrierte AGPL können verwendet werden. Erfindungsgemäße AGPL sind auch Lyso-Acylglycerophospholipide, die sich von AGPL mit zwei Acylresten durch das Fehlen eines Acylrestes in der sn-1- oder *sn*-2-Position unterscheiden. Eingesetzt werden können auch handelsübliche Gemische von Glycerophospholipiden oder Fraktionen solcher Gemische. Ein Beispiel ist das sog. Lecithin, das mindestens 20 % Phosphatidylcholin enthalten muss. Besonders bevorzugt sind AGPL der Formel (II) 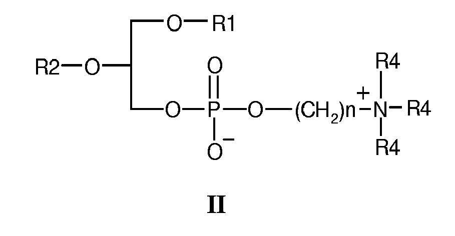 worin
(i) R¹ und R² unabhängig voneinander H oder unverzweigte und unsubstituierte Alkylcarbonylreste sind, welche entweder gesättigt und dann bevorzugt ausgewählt sind aus Lauryl-, Myristyl-, Palmitoyl-, Stearyl-, Arachinyl-, Behenyl- und Lignocerylresten und ganz besonders bevorzugt aus Myristyl-, Palmitoyl-, Stearyl- und Arachniylresten, oder ungesättigt und dann bevorzugt ausgewählt sind aus Oleyl-, α-Linolenyl-, Eicosapentaenyl- und Docosahexaenyl-Resten;
(ii) R⁴ CH₃ oder H ist; und
(iii) n 2 oder 3 ist.
Ganz besonders bevorzugt ist Phosphatidylcholin (PC) mit gesättigten Fettsäureresten, insbesondere Dipalmitoylphosphatidylcholin (DPPC). DPPC ist unter anderem in Lecithin, vor allem in hydriertem Lecithin enthalten. Unter anderem daher ist auch Lecithin, bevorzugt hydriertes Lecithin, für den erfindungsgemäßen Einsatz geeignet. Ein besonders geeignetes Lecithin ist Ei- oder Soja-Lecithin bzw. hydriertes Ei- oder Soja-Lecithin.

Ist das AGPL ein Lyso-PL, so ist die Fettsäure bevorzugt
(i) eine Fettsäure mit einer Länge von mindestens C20,
(ii) eine gesättigte Fettsäure, oder
(iii) eine ω-3 oder ω-9 Fettsäure, die bevorzugt mindestens eine Länge von C18, besonders bevorzugt von C20 hat und insbesondere Eicosapentaensäure (20:5) oder Docosahexaensäure (22:6) ist.

"Wirkstoffe" im Sinne der vorliegenden Erfindung sind Verbindungen, die in Lebewesen, insbesondere in Mensch oder Tier, eine physiologische Reaktion hervorrufen können. Sie sind insbesondere in der Therapie eingesetzte Wirkstoffe. Unter einem "pharmakologisch aktiven Wirkstoff" versteht man eine Verbindung, die als Bestandteil eines Arzneimittels die Ursache für dessen Wirksamkeit ist.

Ein "Triglycerid" ist ein Triester des Glycerins mit gleichen oder unterschiedlichen, gesättigten oder ungesättigten Acylresten mit 10 bis 30 G Atomen. Bevorzugte Triglyceride zur erfindungsgemäßen Verwendung in Kombination mit den AGPL sind gesättigte Fettsäuren und diejenigen ungesättigten Fettsäuren, welche in vivo nicht zu Eicosanoiden, insbesondere nicht zu den biologisch hochwirksamen Eicosanoiden der 2er-Serie wie z. B. PGE₂ umgesetzt werden können. Beispiele hierfür sind gesättigte/hydrierte Triglyceride, MCT, Fischöle bzw. Öle aus der Mikroalge Ulkenia (Ärztezeitung vom 26.8.2004), welche beide sehr viel EPA und DHA enthalten, Olivenöle, Rapsöle, Nachtkerzenöle oder Lein(samen)öle.

In einer bevorzugten Ausführungsform von (1) ist das AGPL das einzige im Medikament vorhandene Phospholipid, besonders bevorzugt das einzige vorhandene GPL. Im letzteren Fall wiederum bevorzugt ist, dass nur Acylgylcerophosphocholin vorhanden ist.

Das AGPL kann als einziger Wirkstoff im Medikament vorhanden sein, es kann jedoch auch mit anderen Wirkstoffen kombiniert werden. Im letzteren Falle ist das AGPL bevorzugt der einzige Wirkstoff aus der Verbindungsklasse der Lipide, insbesondere aus der Klasse der Phospholipide. Das Acylglycerophospolipid wird in einer besonders bevorzugten Ausführungsform jedoch als einziger im Medikament enthaltener pharmakologisch aktiver Wirkstoff eingesetzt.

Wichtig im Zusammenhang mit der vorliegenden Erfindung ist, dass insbesondere bei metastasierenden Tumoren, die häufig Kachexie induzieren, oft auch ein erhöhter Spiegel des hauptsächlich mit inflammatorischen Prozessen assoziierten lipolytischen Enzyms sPLA2 zu beobachten ist (Ogawa, M. et al., Res. Comm. Chem. Pathol. Pharmacol. 74(2):241-244 (1991)). Es ist beschrieben, dass die proinflammatorischen Zytokine TNFα, IFNγ, IL-1 und IL-6 die sPLA2 induzieren (Pruzanski, W. et al., Biochim. Biophys. Acta 1403(1):47-56 (1998); Kudo, I. und Murakami, M., Prostaglandins Other Lipid Mediat. 68-69:3-58 (2002)).

Ein erhöhter Spiegel an lipolytischer sPLA2 könnte einen erhöhten zellulären Phospholipidabbau zur Folge haben, der zunächst zur Freisetzung von Lyso-Phospholipiden und freien Fettsäuren in Zellmembranen führen würde. sPLA2 spaltet bevorzugt Fettsäuren aus der 2-Postion von Phospholipiden. Häufig ist Arachidonsäure eine der bei der sPLA2-Hydrolyse von Membran-Phospholipiden freigesetzten Fettsäuren.

Der durch eine erhöhte PLA2-Aktivität verursachte Zellmembranabbau hätte signifikante Folgen für den Tumorpatienten. Da die bei der Spaltung von Phosholipiden freigesetzten Lyso-Phospholipide zu einem Grossteil durch Lysophospholipid-Lipasen weiter abgebaut werden, muß die Zellen neue Phospholipide aufbauen. Dies geschieht zum einen durch *de novo* Synthese (via CTP-Phosphocholin-cytidyltransferase, CTT) oder durch Reacylierung von externem Lyso-PC (aus dem Serum). Letzteres ist wahrscheinlich der Hauptsyntheseweg für neue Phospholipide, da hohe Lyso-PC-Konzentrationen, wie sie im Serum vorkommen (ca. 300 µM) (Raffelt, K. et al., NMR Biomed. 13(1):8-13 (2000); Sullentrop, F. et al., NMR Biomed. 15(1):60-68 (2000)), CTT inhibieren (Boggs, K. P. et al., J. Biol. Chem. 270(13):7757-64 (1995)).

Eine Folge wäre, dass die beim Phosholipidabbau freigesetzten Fettsären zu Eicosanoiden umgewandelt werden. Damit hätte eine erhöhte sPLA2-Aktivität eine erhöhte Eicosanoidproduktion der Zellen zur Folge, wobei dieses Eicosanoid bei Tumorzellen oft PGE₂, hergestellt aus Arachidonsäure, ist. PGE₂ wiederum wirkt autokrin auf die Tumorzellen und stimuliert deren Wachstum. Zudem ist ein erhöhter PGE₂-Spiegel mit einer erhöhten Metastasierungsrate assoziiert (Attiga, F. A. et al., Cancer Res. 60(16):4629-4637 (2000)). Eine weitere wichtige Implikation eines erhöhten PGE₂-Spiegels ist zudem die Schmerzsensibilisierung bei Tumorpatienten, da PGE₂ die Erregbarkeit von Nozizeptoren steigert (Brune, K. und Zeilhofer, H.U., Biospektrum 1 :36-38 (2004)).

Eine weitere Folge des Phospholipidabbaus könnte eine Depletierung von Lyso-PCs im Serum sein, falls diese verstärkt für eine PL-Neusynthese benötigt werden. Eine solche Depletion könnte schädlich für andere Zellen sein, die auch auf Lysophospholipide angewiesen sind wie z. B. die Zellen des Immunsystems. Dies könnte insbesondere ein Problem bei solchen Patienten darstellen, die am cancer anorexia-cachexia syndrome leiden und nicht mehr ausreichend Nahrung für die Wiederherstellung des Lyso-PC-Spiegels aufnehmen können. Ein Hinweis hierauf ist, dass der Lyso-PC-Spiegel bei kachektischen Tumorpatienten sinkt (Gruppe 3 in Bsp. 4, Fig. 4).
Somit ist die erfindungsgemäße Wirkung der AGPL auf Krebs im allgemeinen und insbesondere auf das Tumorwachstum, sowie insbesondere auf die Krebs-Begleiterscheinungen Tumorkachexie, Metastasierung, Entzündungen und Schmerzempfinden möglicherweise darin begründet, dass die AGPL den beschleunigten zellulären Phospholipid-Abbau und die damit verbundenen oben beschriebenen Folgen wie die Freisetzung von Arachidonsäure und die Aktivierung von sPLA2 reduzieren.

Die erfindungsgemäße Wirkung des AGPL beruht des weiteren vermutlich darauf, dass es *in vivo* zumindest teilweise in Fettsäuren und Lyso-Phospholipide gespalten wird. Diese beiden Komponenten entfalten dann einzeln (parallel) oder gemeinsam eine Wirkung, welche allein durch die Verabreichung einer der beiden Komponenten nicht problemlos erzielt werden könnte. Im Gegenteil könnte die Gabe von Lyso-AGPL wegen seiner hämolytischen Eigenschaften und der gastrointestinalen Nebenwirkungen sogar schaden, während freie Fettsäuren allein andererseits völlig andere pharmakokinetische und biophysikalische Eigenschaften als AGPL besitzen und daher deren Wirkung im Organismus nicht erzielen könnten.

Eine ganz besonders bevorzugte Ausführungsform von (1) ist die erfindungsgemäße Verwendung des AGPL in Kombination mit einen Triglycerid oder den aus einem Triglycerid herstellbaren freien Fettsäuren, Mono- und Diglyceriden. Hiervon ist die Verwendung des Triglycerids als weiterer Bestandteil des Medikaments bevorzugt. Besagte Verbindungen sind oder enthalten bevorzugt überwiegend oder ausschliesslich hydrierte, ω-3 oder ω-9 Fettsäuren, und sie enthalten nicht oder nur in geringen Anteilen ω-6 Fettsäuren. Mit anderen Worten: sie enthalten oder sind überwiegend oder ausschließlich Fettsäurereste, die *in vivo* nicht zu den biologisch hochaktiven Eicosanoiden umgesetzt werden können. Solche Triglyceriden bzw. die resultierenden Di- und Monogylceride und Fettsäuren sind z. B. gesättigte/hydrierte Triglyceride, MCT, Fischöle bzw. Öle aus der Mikroalge Ulkenia (Ärztezeitung vom 26.8.2004), welche beide sehr viel EPA und DHA enthalten, Olivenöle, Rapsöle, Nachtkerzenöle oder Lein(samen)öle.

Dadurch kann die Wirkung des Medikaments, insbesondere die Wirkung auf die tumorinduzierte Kachexie verstärkt werden. Ursache dieses Effekts ist einerseits die bekannte Wirkung von Triglyceriden als hochenergetisches Lebensmittel und Energielieferant. Andererseits ergibt sich aber aus der gleichzeitigen Anwesenheit der Triglyceride und der AGPL ein zusätzlicher Effekt dadurch, dass die *in vivo* teilweise zu Lyso-PL gespaltenen AGPL durch Umesterung einen Fettsäurerest aus den Triglyceriden bzw. den daraus resultierenden Di- und Mono-Glyceriden oder freien Fettsäuren übernehmen können, wodurch dieser Fettsäurerest nun leichter in Zellmembranen eingebaut und nicht primär der Energieversorgung (Fettsäureoxidation) oder der Einlagerung als Triglycerid in Adipocyten zugeführt wird. Sind die in die Zellmembranen eingebauten Fettsäuren überwiegend hydrierte, ω-3 oder ω-9 Fettsäuren, so können die Zellen möglicherweise weniger PGE₂ herstellen. Dies wiederum kann zu einer Verringerung des Tumorwachstums und der Metastasierung, einer Reduktion des Schmerzempfindens und einer Verbesserung der kachektischen Situation führen.

Ausserdem stellen die Triglyceride ein zusätzliches Reservoir an Fettsäuren zur Verfügung, die nicht zu Eicosanoiden umgesetzt werden könne. Dies kann den Teil der Kachexie, welcher durch Eicosanoide verursacht wird, beeinflussen und dadurch die Kachexie reduzieren.

Wesentlich bei der erfindungsgemäßen Verwendung des AGPL in Kombination mit einem oder mehreren Triglyceriden ist, dass der AGPL-Anteil am Gesamt-Lipid-Gehalt des Medikaments hoch ist. So sollte dieser AGPL-Anteil mindestens 10 Gew-% betragen, vorzugsweise jedoch mindestens 20 Gew.-%, ganz besonders bevorzugt mindestens 40 Gew-% des Gesamtlipidgewichts. Dies ist gleichbedeutend mit einem Verhältnis AGPL:Triglycerid von 1 : 9, bevorzugt von 2 : 8, besonders bevorzugt von 4 : 6. Grund herfür ist, dass die AGPL als Wirkstoffe durch die Anwesenheit der Triglyceride in ihrer Wirkung lediglich unterstützt werden. Dies unterscheidet die Medikamente der vorliegenden Erfindung auch von Lipidpräparationen zur künstlichen Ernährung, in denen der PL-Anteil so gering wie möglich gehalten werden muss. Des Weiteren können die AGPL in Kombinantionen mit Triglyceriden ein weites Spektrum von Fettsäuren (hydriert, ungesättigt) enthalten und aus unterschiedlichsten Quellen stammen (Ei, Soja, Fisch etc.), sie sind also nicht wie die Zubereitungen zur künstlichen Ernährung auf Ei-Lecithin beschränkt.

Eine weitere ganz besonders bevorzugte Ausführungsform ist die erfindungsgemäße Verwendung des AGPL in Kombination mit Substanzen, die eine Wirkung auf die Ecosanoidsynthese haben und so die Wirkung des AGPL synergistisch unterstützen können. Erstens sind dies PLA2-Inhibitoren, insbesondere Inhibitoren der sPLA2 (TypII-sPLA, wie sie z. B. in Uhl et al., Phospholipase A2 Basic and Clinical Aspects in Inflammatory Diseases Vol. 24, Karger, Basel, S. 123-175 (1997) und in DE-A-4234130 beschrieben sind.

Zweitens sind dies Substanzen, die die Cyclooxygenasen 1, 2 oder 3 inhibieren, wie die unspezifischen Verbindungen Acetylsalicylsäure, Paracetamol, Diclofenac, Ibuprofen, Metamizol, Phenazon, Propyphenazon sowie COX-2-Inhibitoren, z. B. Meloxicam (Mobec^{®}), Celecoxib (Celebrex^{®}), Rofecoxib (Vioxx^{®}), Etoricoxib (Arcoxia^{®}), Valdecoxib (Rayzon^{®}), und Parecoxib (Dynastat^{®}). Diese Substanzen werden in Konzentrationen/Dosen eingesetzt, wie sie für die therapeutische Verwendung der Substanzen üblich bzw. vom Hersteller vorgeschrieben sind.

Das erfindungsgemäße Medikament ist zur systemischen Gabe geeignet, besonders zur oralen (p.o.) oder intravenösen (i.v.) Gabe. Die orale Gabe ist bevorzugt, insbesondere falls das Medikament Lyso-AGPL enthält.

Die bekannten Nebenwirkungen von Phospholipiden auf das Blutbild bei i.v. Applikation werden bei Einsatz des erfindungsgemäßen Medikaments verhindert, wenn 1,2-Diacylglycerophospholipide verwendet werden. Diese Substanzen sind keine Mizellbildner (Detergenzien), sondern Membranbildner, weswegen sie keine hämolytischen Eigenschaften aufweisen. Daher werden bei intravenöser Anwendung bevorzugt 1,2-Diacylglycerophospholipide verwendet.

Der erfindungsgemäße Anteil des Acylglycerophospholipids an den Gesamtlipiden im Medikament gemäß (1) beträgt mindestens 10 %, bevorzugt mindestens 40 %, ganz besonders bevorzugt 90 - 100 %. Insbesondere kann der AGPL-Anteil 100% betragen, das AGPL ist dann das einzige Lipid im Medikament.

Das erfindungsgemäße Medikament kann wie für lipidhaltige Medikamente üblich formuliert sein, z. B. als Liposomen und liposomale Formulierungen, als übliche Emulsion, als Tabletten, Kapseln oder als Pulver zum Einrühren in Nahrungsmittel. In der bevorzugten Formulierung als Tablette kann die Konzentration der AGPL bis zu 100 % betragen. Weiterhin bevorzugt ist die Formulierung als Liposomen, wobei die AGPL ein Teil der Liposomenmembran sind, in welcher aber auch noch Nicht-AGPL eingebaut sein können. Beispiele für derartige Nicht-AGPL sind Cholesterol und negativ oder positiv geladene Amphiphile (z. B. DOTAP).

Die Dosierung des Medikaments wird von dem behandelnden Arzt individuell auf den jeweiligen Patienten angepasst. Sie richtet sich u. a. nach der Art der Krankheit, der Schwere der zu behandelnden Symptome, der konstitutionellen Beschaffenheit des Patienten usw., wobei üblicherweise Dosen von 2-300 mg/kg Körpergewicht/Tag in Betracht kommen.

Ein besonderer Vorteil der Ausführungsform (1) liegt in ihrer Bedeutung für die Herstellung von Medikamenten zur Therapie von Tumorkachexie.

So besitzt Dipalmitoylphosphatidylcholin Wirkung auf tumorinduzierte Kachexie. Gezeigt wird diese Wirkung in Beispiel 1 anhand des Gewichtsverlaufs von Nacktmäusen, die ein humanes Nierenzellkarzinom (RXF 486) tragen. Dieser Tumor wirkt kachexieinduzierend. Durch orale Gabe eines synthetischen PC mit hydrierten Fettsäuren (Dipalmitoylphosphatidylcholin, DPPC) als liposomale Formulierung in Kochsalzlösung konnte der Gewichtsverlust im Vergleich zu einer unbehandelten Kontrollgruppe praktisch gestoppt werden (Fig. 1).

Ein weiterer bevorzugter Aspekt der Ausführungsform (1) ist die Herstellung von Medikamenten zur Therapie von Tumoren in Mensch und Tier. Bevorzugt sind dies Tumore, für die eine verstärkte sPLA2-Expression beobachtet wurde, also insbesondere Tumore des Gastrointestinaltraktes, des Brustkrebses, des Ovarialkarzinoms, des Pankreaskarzinoms, des Lungenkarzinoms und vor allem des Prostatakarzinoms (Ogawa, M., in: Uhl, W. et al., Phospholipase A2 Basic and Clinical Aspects in Inflammatory Diseases Vol. 24, Karger, Basel, S.200-204 (1997); Yamashita, S. et al., Clin. Chim. Acta 228(2):91-99 (1994); Yamashita, S. et al., J. Cancer 69(6):1166-1170 (1994); Graff, J.R. et al., Clin. Cancer Res. 7(12)3857-3861 (2001)). Weitere und besonders bevorzugte Anwendungsgebiete sind die Therapie von Weichteilsarkomen und Nierenzellkarzinomen.

So wirkt hydriertes PC auf die Tumorprogression bei einem humanen Weichteilsarkom auf der Nacktmaus. Dieser Tumor wirkt nicht kachexieinduzierend. Durch intravenöse Gabe von hydriertem Lecithin als Iiposomale Formulierung mit Cholesterol konnte eine Verlangsamung des Tumorwachstums erreicht werden (Beispiel 2; Fig. 2).

Auch in einem orthotopen Tumormodell ist eine Reduktion der tumorinduzierten Kachexie verbunden mit einer Reduktion des Tumorwachstums durch Gabe von hydriertem Ei-PC nachweisbar (Beispiel 3; Fig. 3).

Die Erfindung wird anhand der nachfolgenden Beispiele näher erläutert, die jedoch die Erfindung nicht einschränken.

### Beispiele

### Beispiel 1: Wirkung von hydriertem PC auf den Gewichtsverlauf von Nacktmäusen, die ein humanes Nierenzellkarzinom (RXF486, tragen

Tumore aus dem humanen Nierenzellkarzinom RXF 486 wirken stark kachexieinduzierend.

Es wurden weibliche thymusaplasierte BALB/c Nacktmäuse (nu/nu) (Charles River, Frederick, Md.) im Alter von 8 bis 10 Wochen eingesetzt. Die Tiere hatten ein Gewicht von 21-23 g und wurden unter einem natürlichen Tag/Nacht-Zyklus gehalten. Sie erhielten Wasser und Nagerfutter (Altromin, Lage, Deutschland) *ad libitum.*

3-5 mm (Durchmesser) große Fragmente des humanen Nierenzellkarzinoms RXF 486 wurden subkutan zwischen die Vorder- und Hinterflanke der Tiere implantiert. Die Therapieexperimente wurden begonnen, wenn die Tumore eine Größe von 30-40 mm³ erreicht hatten.

Zur Therapie wurde synthetisches PL (Dipalmitoylphosphatidylcholin, DPPC; Sygena, Liestal, Schweiz) eingesetzt. Es wurde in einer Konzentration von 33 mg/ml in 0,9 % (w/v) Kochsalzlösung dispergiert (Ultraschalldispergierung in 50 ml Falcon-Tube), so dass eine heterogene liposomale Dispersion entstand. Für die oral Applikation (p.o.) wurde die Dispersion mit Kochsalzlösung auf 3,3 mg/ml eingestellt.

Die Tiere wurden an den Versuchstagen 0 - 4, 7 - 11, 14, 17 und 18 mit jeweils 33 mg/kg Körpergewicht DPPC p.o. per Schlundsonde behandelt (Applikationsvolumen: 10 ml/kg). Die Kontrolltiere erhielten nur Kochsalzlösung. Das Gewicht der Tiere wurde an den Tagen 0, 2, 4, 7, 9, 11, 14, 18, 21, 25, 26 und 29 bestimmt und ist in Fig. 1 in % des Gewichts des Tages 0 dargestellt.

Ergebnis: Durch orale Gabe des DPPC als liposomale Dispersion in Kochsalzlösung konnte der Gewichtsverlust im Vergleich zu einer unbehandelten Kontrollgruppe praktisch gestoppt werden (Fig. 1).

### Beispiel 2: Wirkung von hydriertem PC auf die Tumorprogression bei einem humanen Weichteilsarkom auf der Nacktmaus

Tumore aus dem humanen Weichteilsarkom SXF 1301 wirken nicht kachexieinduzierend.

Es wurden weibliche thymusaplasierte BALB/c Nacktmäuse (nu/nu) (Charles River, Frederick, Md.) im Alter von 8 bis 10 Wochen eingesetzt. Die Tiere hatten ein Gewicht von 21 - 23 g und wurden unter einem natürlichen Tag/Nacht-Zyklus gehalten. Sie erhielten Wasser und Nagerfutter (Altromin, Lage, Deutschland) *ad libitum.*

3 - 5 mm (Durchmesser) große Fragmente des humanen Weichteilsarkoms SXF 1301 wurden subkutan zwischen die Vorder- und Hinterflanke der Tiere implantiert. Die Therapieexperimente wurden begonnen, wenn die Tumore eine Größe von 150 - 400 mm³ erreicht hatten.

Hydriertes Ei-Lecithin (Ei-Phosphatidylcholin, Ei-PC; EPC-3 der Fa. Lipoid, Ludwigshafen, Deutschland) wurde als liposomale Formulierung mit Cholesterol (Merck, Darmstadt, Deutschland) in Phosphatpuffer (20 mM Phosphat, 130 mM NaCl, pH 7,4) in einer Dosis von 840 mg/kg wöchentlich über drei Wochen hinweg durch Injektion in die Schwanzvene von 6 Tieren gegeben (Tage: 0, 7, 14). Das molare Verhältnis Lecithin:Cholesterol in der liposomalen Formulierung betrug 55 : 45, die Liposomen hatten eine Größe von 40 - 60 nm. Das Injektionsvolumen war 10 ml/kg, die verabreichte Gesamtlipidmenge betrug 2,17 mmol/kg. Kontrolltiere (n = 6) erhielten an den Tagen 0, 7 und 14 nur 0,9 % (w/v) Kochsalzlösung.

Die Tumorvolumina wurden durch Calipervermessung (Länge x Breite²) der subkutanen (tastbaren und vermessbaren) Tumore an den Tagen 0, 3, 7, 10, 14, 17, 21, 24 und 28 bestimmt.

Ergebnis: Es konnte durch Gabe des hydrierten Lecithins in der Dosis von 840 mg/kg/Woche i. v. eine Verlangsamung des Tumorwachstums im Vergleich zur Kontrollgruppe um den Faktor 2 erreicht werden. Das Ergebnis ist in Fig. 2 dargestellt.

### Beispiel 3: Wirkung von hydriertem PC auf den Gewichtsverlauf und das Tumorvolumen bei immunkompetenten Mäusen, die ein Nierenzellkarzinom (RENCA-Modell, orthotopes Modell) tragen

### Der RENCA-Tumor wirkt stark kachexieinduzierend.

Es wurden weibliche BALB/c-Mäuse (Charles River, Frederick, Md.) im Alter von 6 bis 8 Wochen eingesetzt. Die Tiere hatten ein Gewicht von ca. 20 g und wurden unter einem natürlichen Tag/Nacht-Zyklus gehalten. Sie erhielten Wasser und Nagerfutter (Altromin, Lage, Deutschland) *ad libitum.*

RENCA-Tumorzellen (murines Nierenzellkarzinom) wurden bei den Tieren operativ durch Injektion in die Niere (genauer: in den subcapsularen Spalt in der linken Niere) eingebracht. Eine Woche nach Instillation von 10⁶ RENCA-Tumorzellen war der Tumor bereits makroskopisch sichtbar und es wurde mit der Behandlung der Mäuse begonnen (Tag 1). Die Tiere hatten zu diesem Zeitpunkt ein Gewicht von 16 - 23 g.

Zur Therapie wurde hydriertes Ei-Lecithin (Ei-Phosphatidylcholin, EPC-3; Fa. Lipoid, Ludwigshafen) eingesetzt. Es wurde in physiologischer Kochsalzlösung durch Ultraschall bei ca. 50 °C dispergiert. Es entstand eine heterogene liposomale Dispersion, die mit Ringerlösung auf 5 mgEPC-3/ml eingestellt wurde.

Den Tieren wurden 10 ml/kg der EPC-3-Dispersion (entsprechend 50 mg/kg EPC-3) per Schlundsonde p. o. an den Tagen 1 - 5, 8 - 12, 15 - 20 verabreicht. Die Kontrollgruppe wurde nichts verabreicht.

Das Gewicht der Tiere wurde an den Tagen 1 , 3, 6, 8, 10, 13, 15, 17 und 20 gemessen. Das Tumorgewicht wurde am Tag 20 nach Tötung der Tiere durch Wiegen der Niere bestimmt. Da es sich um ein orthotopes Tumormodell handelt und Ort des Tumors die Niere war, wurde diese Messung am Ende des Experimentes vorgenommen. Das Körpergewicht an Tag 20 wurde um das Tumorgewicht korrigiert und ist in Fig. 3A wiedergegeben.

Ergebnis: Durch orale Gabe von 50 mg/kg/Tag hydriertem Ei-PC als liposomale Formulierung in Ringerlösung konnte der Gewichtsverlust im Vergleich zu einer unbehandelten Kontrollgruppe signifikant vermindert werden (p: > 0,017; Fig. 3A).

Des weitere resultierte die EPC-3-Behandlung der RENCA-Mäuse nach 20 Tagen in einem Trend zu geringerem Tumorgewicht (p: 0,227; Fig. 3B).

### Beispiel 4: Bestimmung des Lyso-Phosphatidylcholin-Spiegels bei Kachexie-Patienten

Ausgewählt für diese Studie wurden 30 Patienten mit unterschiedlichen soliden Tumoren. Die Patienten hatten entweder keinen Gewichtsverlust seit der Erstdiagnose (n: 11) oder aber einen Gewichtsverlust von < 10 % (n: 10) bzw. einen Gewichtsverlust von > 10 % (n: 9). Im Serum dieser Patienten wurde die Konzentration von Lyso-Phosphatidylcholin (Lyso-PC) mit Hilfe der HPTLC (hochauflösende Dünnschichtchromatograpie) bestimmt. Hierzu wurden die Seren (500 µl) mit 500 µl 0,9%iger Kochsalzlösung verdünnt und 3 mal mit je 2ml Choroform/Methanol (2:1, v/v) extrahiert. Die vereinigten organischen Phasen wurden mit Hilfe eines Stickstoffstromes zur Trockene eingedampft, und der Rückstand in je 300 µl Choroform/Methanol (2:1, v:v) aufgenommen. 50 µl dieser Lösungen wurden mit Hilfe eines Camag Auftrageautomaten auf Silicagel-Platten (für die HPTLC) aufgetragen (Strichlänge 5 mm), ebenfalls aufgetragen wurden entsprechende Lösungen von Lyso-Phosphatidylcholin (E. Merck, Darmstadt) in Chloroform/Methanol (2:1) als Standards. Die Platten wurden in Choroform/Methanol/25%Ammoniak/Wasser (45/30/15/10) entwickelt und anschließend bei 180 °C getrocknet. Die Platten wurden in eine CuSO₄/Phosphorsäure-Lösung getaucht, getrocknet (bei ca. 60 °C) und bei 180 °C gefärbt. Die Intensität der Lyso-PC-Spots wurde mit Hilfe eines Camag-Scanners III quantifiziert und die Konzentration des Lyso-PC's in den Patienten-Seren mit Hilfe der mitaufgetragenen Standards berechnet. Fig. 4 zeigt das Ergebnis der Quantifizierung.

## Patentansprüche

1. Verwendung von einem oder mehreren Acylglycerophospholipiden als Wirkstoff zur Herstellung eines Medikaments zur Therapie von Krebs und Kachexie, Entzündungen und rheumatischen Erkrankungen, Fatigue, Polyneuropathie, multipler Sklerose, sowie zur Therapie der mit diesen Erkrankungen verbundenen Begleiterscheinungen.

2. Verwendung nach Anspruch 1, wobei die Acylglycerophospholipide ausgewählt sind aus 1,2-Diacylglycerophospholipiden, 1-Acylglycerophospholipiden und 2-Acylglycerophospholipiden mit gesättigten oder ungesättigten Acylresten, einschließlich Phosphatidylcholinen, Lyso-Phosphatidylcholinen und Lecithin, und bevorzugt Phosphatidylcholine sind.

3. Verwendung nach Anspruch 1 oder 2, wobei das Acylglycerophospholipid nur gesättigte Acylreste besitzt und insbesondere ein Phosphatidylcholin mit gesättigten Acylresten ist.

4. Verwendung nach einem oder mehreren der Ansprüche 1 bis 3, wobei Verbindungen mit der Struktur der Formel (I) 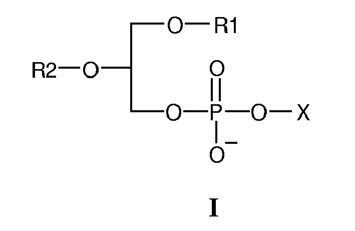 worin
R¹ und R² unabhängig voneinander ausgewählt sind aus H, Alkylcarbonyl-, Alkenylcarbonyl-, Alkinylcarbonyl-, Arylalkylcarbonyl- und Cycloalkylcarbonylresten, worin die Alkylreste geradkettig, verzweigt oder zyklisch, gesättigt oder ungesättigt und mit 1 bis 3 Resten R³ substituiert sein können und in den Alkylresten eines oder mehrere der C-Atome durch O oder NR⁴ ersetzt sein können;
X ausgewählt ist aus H, -(CH₂)ₙ-N(R⁴)₃⁺, -(CH₂)ₙ-CH(N(R⁴)₃⁺)-COO⁻ und -(CH₂)ₙ-CH(OH)-CH₂OH, wobei n eine ganze Zahl von 1 bis 5 ist;
R³ unabhängig vom Auftreten weiterer R³-Reste ausgewählt ist aus H, Niederalkyl (worin die Niederalkylreste geradkettig, verzweigt oder zyklisch, gesättigt oder ungesättigt sein können), F, Cl, CN und OH; und
R⁴ unabhängig vom Auftreten weiterer R⁴-Reste ausgewählt ist aus H, CH₃ und CH₂CH₃,
oder deren pharmazeutisch geeignete Salze verwendet werden.

5. Verwendung nach Anspruch 4, wobei in der Verbindung der Formel (I)
(i) die Alkylcarbonylreste 10 bis 24 C-Atome aufweisen, gesättigt sind oder eine oder mehrere Doppelbindungen enthalten, wobei die Zahl der C-Atome bevorzugt ein Vielfaches von 2 ist und die Doppelbindungen nicht konjugiert sind, und wobei die Alkylreste besonders bevorzugt Fettsäurereste sind;
(ii) die Niederalkylreste 1-3 C-Atome aufweisen und bevorzugt gesättigt sind; und
(iii) n eine ganze Zahl von 1 bis 3 ist.

6. Verwendung nach Anspruch 4 oder 5, wobei in der Verbindung der Formel (I)
(i) R¹ und R² unabhängig voneinander H oder unverzweigte und unsubstituierte Alkylcarbonylreste sind, welche entweder gesättigt sind und dann bevorzugt ausgewählt sind aus Lauryl- (n-Dodecanyl-), Myristyl- (n-Tetradecanyl-), Palmitoyl- (n-Hexadecanyl-), Stearyl- (n-Octadecanyl-), Arachinyl- (n-Eicosanyl-), Behenyl- (n-Docosanyl-) und Lignoceryl- (n-Tetracosanyl-)resten und ganz besonders bevorzugt aus Myristyl-, Palmitoyl-, Stearyl- und Arachniylresten, oder ungesättigt sind und dann bevorzugt ausgewählt sind aus Oleyl- (18:1), α-Linolenyl- (18:3), Eicosapentaenyl- (20:5) und Docosahexaenyl (22:6)-Resten;
(ii) R³ H ist; und
(iii) X -(CH₂-)₂-N(CH₃)₃⁺, -(CH₂-)₂-NH₃⁺, oder -CH₂-CH(NH₃⁺)-COO⁻ ist.

7. Verwendung nach einem oder mehreren der Ansprüche 1 bis 6, wobei das Acylglycerophospholipid synthetisch hergestellt ist oder aus natürlichen Quellen stammt und bevorzugt
(i) Lecithin, besonders bevorzugt hydriertes Lecithin; und/oder
(ii) Dipalmitoylphosphatidylcholin
ist.

8. Verwendung nach einem oder mehreren der Ansprüche 1 bis 7, wobei der Anteil des Acylglycerophospholipids an den Gesamtlipiden im Medikament mindestens 10 Gew-%, bevorzugt mindestens 40 Gew.-%, ganz besonders bevorzugt 100 Gew.-% beträgt.

9. Verwendung nach einem oder mehreren der Ansprüche 1 bis 8, wobei das Acylglycerophospholipid der einzige vorhandene pharmakologisch aktive Wirkstoff ist.

10. Verwendung nach einem oder mehreren der Ansprüche 1 bis 8, wobei das Medikament weiterhin als zusätzliche Bestandteile
(i) Triglyceride, freie Fettsäuren, Diglyceride und/oder Monoglyceride, wobei der Gesamtanteil der genannten Verbindungen vorzugsweise maximal 90 Gew.-% aller im Medikament vorhandene Lipide beträgt, und/oder
(ii) Substanzen mit Wirkung auf den Phospholipidmetabolismus, bevorzugt Cyclooxygenase-Inhibitoren oder Phospholipase-A2-Inhibitoren,
enthält.

11. Verwendung nach Anspruch 10, wobei der zusätzliche Bestandteil des Medikaments ein oder mehrere Triglyceride sind, und wobei bevorzugt das Verhältnis AGPL : Triglycerid gleich oder größer 1 : 9 ist.

12. Verwendung nach einem oder mehreren der Ansprüche 1 bis 11, wobei das Medikament
(i) zur oralen oder intravenösen Gabe geeignet ist und bevorzugt oral verabreicht wird; und/oder
(ii) zur Therapie von Krebs, besonders von Weichteilsarkomen und Nierenzellkarzinomen, sowie von Tumorkachexie geeignet ist.

13. Methode zur Therapie von Krebs und Kachexie, Entzündungen und rheumatischen Erkrankungen, Fatigue, Polyneuropathie, multipler Sklerose, sowie zur Therapie der mit diesen Erkrankungen verbundenen Begleiterscheinungen in einem Patienten, umfassend die Gabe eines wie in einem oder mehreren der Ansprüche 1 bis 12 definierten Medikaments, vorzugsweise in einer Dosis von 2-300 mg/kg und Tag, an den Patienten.
